# EUROPÄISCHE PATENTSCHRIFT

(11) **EP 1 634 902 B1**
(45) Veröffentlichungstag und Bekanntmachung des Hinweises auf die Patenterteilung: **28.12.2016**
(21) Anmeldenummer: 05018640.2
(22) Anmeldetag: 27.08.2005
(51) Int. Cl.: C08G 18/79, C08G 18/78, C08G 18/67, C08G 18/81, C09D 175/14, C09D 175/16

(54) **Niedrigviskose Allophanate mit aktinisch härtbaren Gruppen**
Low-viscosity allophanates having actinically hardenable groups
Allophanates à faible viscosité comprenant des groupes à durcissement actinique

(30) Priorität: 09.09.2004 DE 102004043539
(43) Veröffentlichungstag der Anmeldung: 15.03.2006
(73) Patentinhaber: Allnex IP S.à.r.l., 1660 Luxembourg (LU)
(72) Erfinder: Weikard, Jan, Dr., 51519 Odenthal (DE); Richter, Frank, Dr., 51373 Leverkusen (DE); Gürtler, Christophe, Dr., 50676 Köln (DE); Fischer, Wolfgang, Dr., 40668 Meerbusch (DE); Schmitz, Jörg, Dr., 51065 Köln (DE); Mundstock, Holger, 42929 Wermelskirchen (DE)
(74) Vertreter: Destryker, Elise Martine

(56) Entgegenhaltungen:
- EP-A- 1 475 399
- WO-A-00/39183
- DE-A1- 10 205 608

## Beschreibung

Die vorliegende Erfindung betrifft niedrigviskose Umsetzungsprodukte von Alkoholen, die aktivierte unter Einwirkung aktinischer Strahlung mit ethylenisch ungesättigten Verbindungen unter Polymerisation reagierende Gruppen enthalten, mit Polyisocyanaten ein Verfahren zu ihrer Herstellung sowie deren Verwendung in Beschichtungsmitteln.

Die Härtung von aktivierten Doppelbindungen tragenden Beschichtungssystemen durch aktinische Strahlung, wie z. B. UV-Licht, IR-Strahlung oder auch Elektronenstrahlung ist bekannt und technisch etabliert. Es ist eine der schnellsten Härtungsmethoden in der Beschichtungstechnologie. Auf diesem Prinzip basierende Beschichtungsmittel werden daher als strahlen- oder aktinisch- härtende bzw. härtbare Systeme bezeichnet.

Bedingt durch die ökologischen und ökonomischen Anforderungen an moderne Lacksysteme möglichst wenig oder gar kein organisches Lösungsmittel zur Viskositätseinstellung zu verwenden, besteht der Wunsch, bereits niedrigviskose Lackrohstoffe zu verwenden. Bekannt hierfür sind seit langem Polyisocyanate mit Allophanatstruktur wie sie u. a. in EP-A 0 682 012 beschrieben werden.

In der Technik werden diese durch Umsetzung eines ein- oder mehrwertigen Alkohols mit überschüssigem aliphatischen und/oder cycloaliphatischen Diisocyanat hergestellt (vgl. GB-A 994 890, EP-A 0 000 194 oder EP-A 0 712 840). Anschließend erfolgt die Entfernung nicht reagierten Diisocyanats mittels Abdestillation im Vakuum. Nach DE-A 198 60 041 kann diese Verfahrensweise auch mit OH-funktionellen Verbindungen mit aktivierten Doppelbindungen, wie z.B. Hydroxyalkylacrylaten, durchgeführt werden, wobei jedoch zur Herstellung besonders monomerenarmer Produkte Schwierigkeiten auftreten. Da der Destillationsschritt bei Temperaturen bis zu 135°C verlaufen muss, um den Rest-Isocyanatgehalt ausreichend senken zu können (< 0,5 Gew.-% Restmonomer), können während der Aufreinigung bereits Doppelbindungen thermisch initiiert unter Polymerisation reagieren, so dass keine einwandfreien Produkte mehr erhalten werden.

Die Herstellung monomerenarmer, allophanathaltiger, strahlenhärtender Bindemittel auf Polyurethanbasis wird in EP-A 0 867 457 und US-A 5 739 251 beschrieben. Diese Bindemittel tragen allerdings keine aktivierten Doppelbindungen, sondern reaktionsträge Allylethergruppen (Struktur R-O-CH₂-CH=CH₂). Daher benötigt man den Zusatz von Reaktivverdünnern (niedermolekulare Ester der Acrylsäure), die die notwendige UV-Reaktivität einbringen.

EP-A 0 825 211 beschreibt ein Verfahren zum Aufbau von Allophanatstrukturen aus Oxadiazintrionen, wobei allerdings keine strahlenhärtenden Derivate mit aktivierten Doppelbindungen bekannt sind.

Lediglich die Verwendung von Maleinat- und/oder Fumarat-haltigen Polyestern wird erwähnt, die Möglichkeit der Strahlenhärtung wird nicht beschrieben.

US-A 5 777 024 beschreibt die Herstellung der niedrigviskosen strahlenhärtenden Allophanaten durch eine Reaktion von hydroxyfunktionellen Monomeren, die aktivierte Doppelbindungen tragen, mit Isocyanatgruppen von allophanatmodifizierten Isocyanurat-Polyisocyanaten. Die über die Allophanatgruppen gebundenen Reste sind dabei gesättigt.

Die Bildung von Allophanat-Verbindungen durch Ringöffnung von Uretdionen mit Alkoholen ist als Vernetzungsmechanismus in Pulverlacken grundsätzlich bekannt (vgl. Proceedings of the International Waterborne, High-Solids, and Powder Coatings Symposium 2001, 28th, 405-419 sowie US-A 2003 0153713). Allerdings sind die dazu nötigen Reaktionstemperaturen für eine gezielte Herstellung von strahlenhärtenden Monomeren auf Allophanatbasis mit aktivierten Doppelbindungen zu hoch (> 130°C).

Historisch wurde die direkte Reaktion von Uretdion-Ringen mit Alkoholen zu Allophanaten erstmals für lösemittelhaltige isocyanatfreie 2K-Polyurethan-Lacke untersucht. Unkatalysiert ist diese Reaktion aufgrund der geringen Reaktionsgeschwindigkeit ohne technische Bedeutung (F. Schmitt, Angew. Makromol. Chem. (1989), 171, S. 21-38). Mit geeigneten Katalysatoren soll die Vernetzungsreaktion zwischen HDI-basierten Uretdionhärtern und Polyolen aber schon bei 60 bis 80°C einsetzen (K. B. Chandalia; R. A Englebach; S. L.Goldstein; R. W. Good; S. H. Harris; M. J. Morgan; P. J. Whitman; R. T. Wojcik, Proceedings of the International Waterborne, High-Solids, and Powder Coatings Symposium, (2001), S. 77 - 89). Die Struktur dieser Katalysatoren wurde bisher nicht veröffentlicht. Kommerzielle Produkte, zu deren Herstellung diese Reaktion genutzt wird, sind bisher auch nicht bekannt.

EP1475399A1 betrifft feste uretdiongruppenhaltige Polyurethan-Pulverlackzusammensetzungen, enthaltend mindestens einen Katalysator der Formel [NR¹R²R³R⁴]⁺ [R⁵COO]⁻, wobei R¹- R⁴ gleichzeitig oder unabhängig voneinander Alkyl-, Aryl-, Aralkyl-, Heteroaryl-, Alkoxyalkylreste, jeweils linear oder verzweigt, unverbrückt oder mit anderen Resten R¹- R⁴ verbrückt, unter Ausbildung von Cyclen, Bicyclen oder Tricyclen und die Verbrückungsatome neben Kohlenstoff auch Heteroatome sein können, mit 1-18 Kohlenstoffatomen bedeuten und jeder Rest R¹- R⁴ zusätzlich noch eine oder mehrere Alkohol-, Amino-, Ester-, Keto-, Thio-, Urethan-, Harnstoff, Allophanatgruppen, Doppelbindungen, Dreifachbindungen oder Halogenatome aufweisen kann, und R⁵ ein Alkyl-, Aryl-, Aralkyl-, Heteroaryl-, Alkoxyalkylrest, linear oder verzweigt, mit 1-18 Kohlenstoffatomen darstellt und zusätzlich noch eine oder mehrere Alkohol-, Amino-, Ester-, Keto-, Thio-, Urethan-, Harnstoff-, Allophanatgruppen, Doppelbindungen, Dreifachbindungen oder Halogenatome aufweisen kann, die bei niedrigen Einbrenntemperaturen aushärten, Verfahren zur Herstellung derartiger Zusammensetzungen sowie deren Verwendung zur Herstellung von Kunststoffen, insbesondere Pulverlackbeschichtungen, die zu hochglänzenden oder matten, licht- und wetterstabilen Lackfilmen vernetzen.

WO 00/39183 A1 offenbart Verbindungen mit Isocyanatgruppen oder verkappten Isocyanatgruppen, Allophanatgruppen und radikalisch polymerisierbaren C-C-Doppelbindungen, wobei die C-C-Doppelbindungen durch eine direkt daran gebundene Carboxylgruppe oder ein O-Atom in Etherfunktion aktiviert sind (Aktivierte Doppelbindungen), abgeleitet von Polyisocyanaten und Alkoholen A, die neben der Alkoholgruppe noch eine Aktivierte Doppelbindung tragen (Verbindungen (I)), sowie strahlungshärtbare Zubereitungen und Beschichtungsmittel die die Verbindungen (I) enthalten und darauf bewgene Verfahren und Verwendungen.

DE10205608 A1 offenbart feste uretdiongruppenhaltige Polyurethan-Pulverlackzusammensetzungen, die bei niedrigen Einbrenntemperaturen aushärten, Verfahren zur Herstellung derartiger Zusammensetzungen sowie deren Verwendung zur Herstellung von Kunststoffen, insbesondere Pulverlackbeschichtungen, die zu hochglänzenden oder matten, licht- und wetterstabilen Lackfilmen vernetzen.

US 5739251 A offenbart ethylenisch ungesättigte Polyurethan, die Allophanatgruppen enthalten, ungesättigte Isocyanatgruppen-enthaltende Vorläufer dieser Polyurethane und die Verwendung der Polyurethane als Bindemittel in Einkomponenten-Beschichtungszusammensetzungen.

Zusammenfassend lässt sich festhalten, dass die Herstellung von niedrigviskosen strahlenhärtenden Allophanaten durch eine ringöffnende Umsetzung von Alkoholen, die aktivierte Doppelbindungen tragen, mit Uretdionen, unter Einsatz von Ammonium- oder Phosphoniumsalzen aliphatischer oder cycloaliphatischer Carbonsäuren als Katalysatoren bei Temperaturen ≤ 130°C aus dem Stand der Technik her nicht explizit beschrieben wird.

Überraschenderweise wurde nun gefunden, dass aus der Umsetzung von Uretdionen mit olefinisch ungesättigten Alkoholen, die bevorzugt aktivierte Doppelbindungen enthalten, unter Einsatz von Ammonium- oder Phosphoniumsalzen aliphatischer oder cycloaliphatischer Carbonsäuren als Katalysatoren bereits bei Temperaturen ≤ 130°C niedrigviskose strahlenhärtende Allophanate mit niedrigen Restmonomeranteilen erhalten werden können, die bevorzugt Viskositäten gemessen bei 23°C von weniger als 100 000 mPas aufweisen.

Gegenstand der Erfindung ist daher ein Verfahren zur Herstellung strahlenhärtender, allophanatgruppenhaltiger Bindemittel, bei dem bei Temperaturen ≤ 130°C
A) eine oder mehrere uretdiongruppenhaltige Verbindungen mit
B) einer oder mehreren OH-funktionellen Verbindungen, die unter Einwirkung aktinischer Strahlung mit ethylenisch ungesättigten Verbindungen unter Polymerisation reagierende Gruppen (strahlenhärtende Gruppen) aufweisen und
C) gegebenenfalls weiteren gegenüber NCO-Gruppen reaktiven Verbindungen in Anwesenheit
D) einer oder mehrerer Verbindungen enthaltend mindestens ein tetrasubstituertes Ammonium- oder Phosphoniumsalz einer aliphatischen oder cycloaliphatischen Carbonsäure als Katalysator und
E) eines oder mehreren Stabilisatoren und optional Hilfs- und Zusatzstoffen
unter Öffnung des Uretdionringes zu Allophanatgruppen umgesetzt werden, wobei das Verhältnis der OH-Gruppen aus der Komponente B) zu der Summe aus NCO- und Uretdiongruppen aus A) von 1,5 : 1,0 bis 1,0 : 1,9 beträgt; zunächst A) mit B) bis zur vollständigen Reaktion der NCO-Gruppen umgesetzt wird - wobei E) oder Teile davon bereits anwesend sein können - um anschließend durch Zugabe von D) und zusätzlich gegebenenfalls durch Temperaturanpassung die Reaktion der Uretdiongruppen von A) mit B) zu starten; die erhältlichen ungesättigten Allophanate Gehalte an freien Di- bzw. Triisocyanatmonomeren von unter 0,5 Gew.-%, bevorzugt unter 0,1 Gew.-% aufweisen; und die Umsetzung der Komponente A) mit B) und weiteren gegenüber NCO-Gruppen reaktiven Verbindungen C) dann abgeschlossen ist, sobald alle NCO-Gruppen gemäß der gewählten stöchiometrischen Verhältnisse mit NCO-reaktiven Gruppen aus B) und C) abreagiert haben und alle Uretdiongruppen gemäß der gewählten stöchiometrischen Verhältnisse mit den Hydroxylgruppen aus B) abreagiert haben.

Darüber hinaus sind die nach dem erfindungsgemäßen Verfahren erhältlichen Bindemittel ein Gegenstand der Erfindung.

In Komponente A) können alle organischen Verbindungen eingesetzt werden, die mindestens eine Uretdiongruppe aufweisen.

Bevorzugt sind dies Verbindungen, die durch katalytische Dimerisierung von aliphatischen, cycloaliphatischen und/oder araliphatischen Di- oder Polyisocyanaten nach an sich bekannten Verfahren erhältlich sind (vgl. J. Prakt. Chem. 1994, 336, Seite 196 -198).

Geeignete Diisocyanate sind beispielsweise 1,4-Diisocyanatobutan, 1,6-Diisocyanatohexan (HDI), Trimethylhexandiisocyanat, 1,3- und 1,4-bis-Isocyanatomethylcyclohexan, Isophorondiisocyanat (IPDI), 4,4-Diisocyanatodicyclohexylmethane, 1,3- und 1,4-Xylylendiisocyanate (XDI Handelsprodukt der Fa. Takeda, Japan), Diphenylmethan-4,4'-diisocyanat und Diphenylmethan-2,4'-diisocyanat (MDI), 2,4- und 2,6-Toluendiisocyanat (TDI), oder ihre Mischungen. Bevorzugt ist 1,6-Diisocyanatohexan.

Als Katalysatoren kommen dabei z.B.: Trialkylphosphine, Dimethylaminopyridine, Tris-(dimethyl-amino)-phosphin zum Einsatz.

Das Ergebnis der Dimerisierungsreaktion hängt in dem Fachmann bekannter Weise von dem verwendeten Katalysator, den Verfahrensbedingungen sowie den eingesetzten Diisocyanaten ab. Insbesondere können Produkte entstehen, die im Mittel mehr als eine Uretdiongruppe pro Molekül aufweisen, wobei die Anzahl der Urediongruppen einer Verteilung unterliegt. In Abhängigkeit von dem verwendeten Katalysator, den Verfahrensbedingungen sowie den eingesetzten Diisocyanaten entstehen auch Produktgemische, die neben Uretdionen auch andere Struktureinheiten aufweisen wie z.B. Isocyanurat und/oder Iminooxadiazindion.

Besonders bevorzugte Produkte sind durch katalytische Dimerisierung von HDI erhältlich und weisen einen Gehalt an freien HDI von unter 0,5 Gew.-%, einen NCO-Gehalt von 17 bis 25 Gew.-%, bevorzugt von 21 bis 24 Gew.-% und eine Viskosität bei 23°C von 20 bis 500 mPas, bevorzugt von 50 bis 200 mPas auf.

Die durch katalytische Dimerisierung erhältlichen in der Regel NCO-funktionellen Verbindungen werden bevorzugt direkt als Teil von Komponente A) eingesetzt, sie können aber grundsätzlich auch zunächst noch weiter umgesetzt werden und dann erst in A) verwendet werden. Dies kann beispielsweise eine Blockierung der freien NCO-Gruppen oder die weitere Umsetzung von NCO-Gruppen mit NCO-reaktiven zwei- oder mehrfachfunktionellen Verbindungen zu Iminooxadiazindion-, Isocyanurat-, Urethan-, Allophanat-, Biuret-, Harnstoff-, Oxadiazintrion, Oxazolidinon-, Acylharnstoff- oder Carbodiimid-Strukturen sein. Dabei werden höhermolekulare uretdiongruppenhaltige Verbindungen erhalten, die abhängig von der gewählten Verhältnisse NCO-gruppenhaltig oder NCO-gruppenfrei sein können.

Beispielsweise geeignete Blockierungsmittel sind Alkohole, Lactame, Oxime, Malonester, Alkylacetoacetate, Triazole, Phenole, Imidazole, Pyrazole sowie Amine, wie z.B. Butanonoxim, Diisopropylamin, 1,2,4-Triazol, Dimethyl-1,2,4-triazol, Imidazol, Malonsäurediethylester, Acetessigester, Acetonoxim, 3,5-Dimethylpyrazol, ε-Caprolactam, N-tert.-Butyl-benzylamin, Cyclopentanoncarboxyethylester oder beliebige Gemische dieser Blockierungsmittel. Die Verfahrensweise zur Blockierung von NCO-Gruppen ist dem Fachmann vertraut und exemplarisch in Progress in Organic Coatings 1999, 36, 148-172 beschrieben.

NCO-reaktive zwei- oder mehrfachfunktionelle Verbindungen zur Derivatisierung der in A) eingesetzten Uretdione können die oben genannten Di- und/oder Polyisocyanate sein, weiterhin einfache zwei- oder mehrfunktionelle Alkohole wie Ethylenglykol, Propandiol-1,2, Propandiol-1,3, Diethylenglykol, Dipropylenglykol, die isomeren Butandiole, Neopentylglykol, Hexandiol-1,6, 2-Ethylhexandiol und Tripropylenglykol oder auch alkoxylierte Derivate dieser Alkohole. Bevorzugte zweiwertige Alkohole sind Hexandiol-1,6, Dipropylenglykol und Tripropylenglykol. Geeignete dreiwertige Alkohole sind Glycerin oder Trimethylolpropan oder deren alkoxylierte Derivate. Vierwertige Alkohole sind Pentaerythrit oder dessen alkoxylierte Derivate.

Unter aktinischer Strahlung wird elektromagnetische, ionisierende Strahlung verstanden, insbesondere Elektronenstrahlen, UV-Strahlen sowie sichtbares Licht (Roche Lexikon Medizin, 4. Auflage; Urban & Fischer Verlag, München 1999).

Unter Einwirkung aktinischer Strahlung mit ethylenisch ungesättigten Verbindungen unter Polymerisation reagierende Gruppen (strahlenhärtende Gruppen) sind beispielsweise Vinyl-, Vinylether-, Propenyl-, Allyl-, Maleinyl-, Fumaryl-, Maleinimid-, Dicyclopentadienyl-, Acrylamid-, Acryl- und Methacryl-Gruppen, wobei bevorzugt aktivierte Gruppen dieser Art wie Vinylether-, Acrylat- und/oder Methacrylat-Gruppen, besonders bevorzugt Acrylatgruppen in den Verbindungen der Komponente B) zum Einsatz kommen.

Beispielsweise geeignete hydroxylgruppenhaltige Verbindungen der Komponente B) sind 2-Hydroxyethyl(meth)acrylat, Polyethylenoxid-mono(meth)acrylat (z.B. PEA6 / PEM6; Laporte Performance Chemicals Ltd., UK), Polypropylenoxidmono(meth)acrylat (z.B. PPA6, PPM5S; Laporte Performance Chemicals Ltd., UK), Polyalkylenoxidmono(meth)acrylat (z.B. PEM63P, Laporte Performance Chemicals Ltd. , UK), Poly(ε-caprolacton)mono(meth)acrylate wie z.B. Tone M100^{®} (Dow, Schwalbach, DE), 2-Hydroxypropyl(meth)acrylat, 4-Hydroxybutyl(meth)acrylat, Hydroxybutylvinylether, 3-Hydroxy-2,2-dimethylpropyl(meth)acrylat, die hydroxyfunktionellen Mono-, Di- oder soweit möglich höheren Acrylate wie z.B. Glycerindi(meth)acrylat, Trimethylolpropandi(meth)acrylat, Pentaerythrittri(meth)acrylat oder Dipentaerythritpenta(meth)acrylat, die durch Umsetzung mehrwertiger gegebenenfalls alkoxylierter Alkohole wie Trimethylolpropan, Glycerin, Pentaerythrit, Dipentaerythrit zugänglich sind.

Ebenfalls geeignet als Bestandteil von B) sind auch Alkohole, die sich aus der Umsetzung von doppelbindungshaltigen Säuren mit gegebenenfalls doppelbindungshaltigen Epoxidverbindungen erhalten werden, so z.B. die Umsetzungsprodukte von (Meth)acrylsäure mit Glycidyl(meth)acrylat oder Bisphenol-A-diglycidylether.

Darüber hinaus können ebenfalls ungesättigte Alkohole eingesetzt werden, die aus der Umsetzung von gegebenenfalls ungesättigten Säureanhydriden mit gegebenenfalls acrylatgruppenhaltigen Hydroxy- und Epoxidverbindungen erhalten werden. Beispielsweise sind dies die Umsetzungsprodukte von Maleinsäureanhydrid mit 2-Hydroxyethyl(meth)acrylat und Glycidyl(meth)acrylat.

Besonders bevorzugt entsprechen die Verbindungen der Komponente B) der vorgenannten Art und weisen eine OH-Funktionalität von 0,9 bis 1,1 auf.

Ganz besonders bevorzugt werden in B) Verbindungen der vorstehend genannten Art mit primären Hydroxylgruppen eingesetzt. Solche sind bevorzugt 2-Hydroxyethylacrylat und 4-Hydroxybutylacrylat.

Neben den OH-funktionellen ungesättigten Verbindungen der Komponente B) können im erfindungsgemäßen Verfahren auch weitere Verbindungen C) eingesetzt werden, die verschieden von denen aus B) sind und NCO-reaktive Gruppen wie beispielsweise OH, SH oder NH aufweisen.

Dies können beispielsweise NH- oder SH-funktionelle Verbindungen mit unter Einwirkung aktinischer Strahlung mit ethylenisch ungesättigten Verbindungen unter Polymerisation reagierende Gruppen sein.

Weiterhin können hydrophilierend wirkende Gruppen eingebaut werden, insbesondere wenn eine Verwendung aus wässrigem Medium, z.B. in einem wässrigen Lack, vorgesehen ist. Hydrophilierend wirkende Gruppen sind ionische Gruppen, die entweder kationischer oder anionischer Natur sein können und/oder nichtionische hydrophile Gruppen. Kationisch, anionisch oder nichtionisch dispergierend wirkende Verbindungen sind solche, die beispielsweise Sulfonium-, Ammonium-, Phosphonium-, Carboxylat-, Sulfonat-, Phosphonat-Gruppen oder die Gruppen, die durch Salzbildung in die vorgenannten Gruppen überführt werden können (potentiell ionische Gruppen) oder Polyethergruppen enthalten und durch vorhandene isocyanatreaktive Gruppen eingebaut werden können. Bevorzugt geeignete isocyanatreaktive Gruppen sind Hydroxyl- und Amingruppen.

Geeignete ionische oder potentiell ionische Gruppen enthaltende Verbindungen sind z.B. Mono- und Dihydroxycarbonsäuren, Mono- und Diaminocarbonsäuren, Mono- und Dihydroxysulfonsäuren, Mono- und Diaminosulfonsäuren sowie Mono- und Dihydroxyphosphonsäuren oder Mono- und Diaminophosphonsäuren und ihre Salze wie Dimethylolpropionsäure, Dimethylolbuttersäure, Hydroxypivalinsäure, N-(2-Aminoethyl)-ß-alanin, 2-(2-Amino-ethylamino)-ethansulfonsäure, Ethylendiamin-propyl- oder butylsulfonsäure, 1,2- oder 1,3-Propylendiamin-ß-ethylsulfonsäure, Äpfelsäure, Zitronensäure, Glykolsäure, Milchsäure, Glycin, Alanin, Taurin, Lysin, 3,5-Diaminobenzoesäure, ein Additionsprodukt von IPDI und Acrylsäure (EP-A 0 916 647, Beispiel 1) und dessen Alkali- und/oder Ammoniumsalze; das Addukt von Natriumbisulfit an Buten-2-diol-1,4, Polyethersulfonat, das propoxylierte Addukt aus 2-Butendiol und NaHSO₃, z.B. beschrieben in der DE-A 2 446 440 (Seite 5-9, Formel I-III) sowie in kationische Gruppen überführbare Bausteine wie N-Methyl-diethanolamin als hydrophile Aufbaukomponenten. Bevorzugte ionische oder potentielle ionische Verbindungen sind solche, die über Carboxy- oder Carboxylat- und/oder Sulfonatgruppen und/oder Ammoniumgruppen verfügen. Besonders bevorzugte ionische Verbindungen sind solche, die Carboxyl- und/oder Sulfonatgruppen als ionische oder potentiell ionische Gruppen enthalten, wie die Salze von N-(2-Aminoethyl)-ß-alanin, der 2-(2-Amino-ethylamino-)ethansulfonsäure oder des Additionsproduktes von IPDI und Acrylsäure (EP-A 0 916 647, Beispiel 1) sowie der Dimethylolpropionsäure.

Geeignete nichtionisch hydrophilierend wirkende Verbindungen sind z.B. Polyoxyalkylenether, die mindestens eine Hydroxy- oder Aminogruppe enthalten. Diese Polyether enthalten einen Anteil von 30 Gew.-% bis 100 Gew.-% an Bausteinen, die vom Ethylenoxid abgeleitet sind. In Frage kommen linear aufgebaute Polyether einer Funktionalität von 1 bis 3, aber auch Verbindungen der allgemeinen Formel (I), 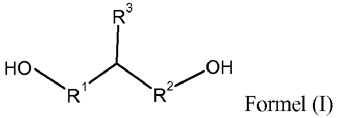 in welcher
- R¹ und R²: unabhängig voneinander jeweils einen zweiwertigen aliphatischen, cycloaliphatischen oder aromatischen Rest mit 1 bis 18 C-Atomen, die durch Sauerstoff und/oder Stickstoffatome unterbrochen sein können, bedeuten und
- R³: für einen alkoxyterminierten Polyethylenoxidrest steht.

Nichtionisch hydrophilierend wirkende Verbindungen sind beispielsweise auch einwertige, im statistischen Mittel 5 bis 70, bevorzugt 7 bis 55 Ethylenoxideinheiten pro Molekül aufweisende Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4.Auflage, Band 19, Verlag Chemie, Weinheim S. 31-38).

Geeignete Startermoleküle sind beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole. Besonders bevorzugt wird Diethylenglykolmonobutylether als Startermolekül verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Bei den Polyalkylenoxidpolyetheralkoholen handelt es sich entweder um reine Polyethylenoxidpolyether oder gemischte Polyalkylenoxidpolyether, deren Alkylenoxideinheiten zu mindestens 30 mol-% bevorzugt zu mindestens 40 mol-% aus Ethylenoxideinheiten bestehen. Bevorzugte nichtionische Verbindungen sind monofunktionelle gemischte Polyalkylenoxidpolyether, die mindestens 40 mol-% Ethylenoxid- und maximal 60 mol-% Propylenoxideinheiten aufweisen.

Insbesondere bei der Verwendung eines ionische Gruppen enthaltenden Hydrophilierungsmittels muss sein Einfluss auf die Wirkung des Katalysators D) überprüft werden. Aus diesem Grund sind nichtionische Verbindungen als Hydrophilierungsmittel bevorzugt.

Als Verbindungen der Katalysatorkomponente D) können neben den erfindungsgemäß zu verwendenden Ammonium- oder Phosphoniumsalzen aliphatischer Carbonsäuren grundsätzlich auch die dem Fachmann an sich bekannten Verbindungen zur Katalyse der Reaktion von Isocyanatgruppen mit isocyanatreaktiven Gruppen einzeln oder in beliebigen Mischungen untereinander verwendet werden.

Beispielhaft sind hier tert. Amine wie Triethylamin, Pyridin, Methylpyridin, Benzyl-dimethylamin, N,N-Endoethylenpiperazin, N-Methyl-piperidin, Pentamethyldiethy-len-triamin, N,N-Dimethylaminocyclohexan, N,N'-Dimethyl-piperazin, 1,4-Diazabicyclo[2.2.2]octan (DABCO) oder Metallsalze wie Eisen(III)-chlorid, Zinn(II)-octoat, Zinn(II)-ethyl-caproat, Zinn(II)-palmitat, Dibutylzinn(IV)-dilaurat, Dibutylzinn(IV)-diacetat und Molybdänglykolat oder beliebige Gemische solcher Katalysatoren zunennen.

Die tetrasubstituerten Ammonium- oder Phosphoniumsalze aliphatischer oder cycloaliphatischer Carbonsäuren der Komponente D) entsprechen bevorzugt der allgemeinen Formel (II), 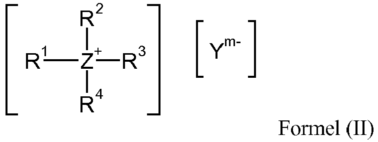 in welcher
- Z: für Stickstoff oder Phosphor steht,
- R¹, R², R³, R⁴: unabhängig voneinander Wasserstoff oder gleiche oder verschiedene aliphatische, cycloaliphatische oder araliphatische Reste mit bis zu 24 Kohlenstoffatomen sind und
- Y: ein Carboxylatrest der allgemeinen Formel (III) ist, 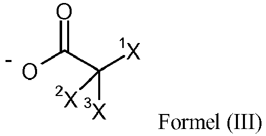
in welcher
¹X, ²X, ³X unabhängig voneinander Substituenten ausgewählt aus der Gruppe bestehend aus Wasserstoff, Halogen, Cyano-, Hydroxy-, Amid-, Ether-, Ester-, Thioether-, Keton-, Aldehyd- und Carboxylatgruppe sowie aliphatische oder cycloaliphatische Reste mit bis zu 24 Kohlenstoffatomen sind, die gegebenenfalls Teile cyclischer oder polycyclischer Systeme sind.

Als tetrasubstituerte Ammonium- oder Phosphoniumsalze aliphatischer oder cycloaliphatischer Carbonsäuren der Formel (II) werden besonders bevorzugt Tetraalkylammoniumcarboxylate, die bevorzugt auf aliphatischen Carbonsäuren mit verzweigten Alkylresten ohne weitere funktionelle Gruppen basieren, eingesetzt.

Besonders bevorzugte Tetraalkylammoniumcarboxylate sind Tetrabutylammonium-2-ethylhexanoat, Tetrabutylammonium-pivalat, Cholin-2-ethylhexanoat, Cholin-pivalat, Methylcholin-2-ethylhexanoat und/oder Methylcholin-pivalat, deren Herstellung in US 5,691,440 beschrieben ist.

In einer bevorzugten Ausführungsform der Erfindung werden in D) ausschließlich Carboxylate der vorstehend genannten Art als einzige Verbindung der Komponente D) verwendet.

Es ist auch möglich die Katalysatoren D) nach dem Fachmann bekannten Methoden auf Trägermaterialien zu bringen und als heterogene Katalysatoren zu verwenden.

Die Verbindungen der Katalysatorkomponente D) können vorteilhafterweise in einer der im Verfahren beteiligten Komponenten oder einem Teil davon gelöst werden. Insbesondere lösen sich die erfindungsgemäß einzusetzenden Carboxylate sehr gut in den polaren Hydroxyalkylacrylaten, so dass D) gelöst in kleinen Mengen von B) als konzentrierte Lösung flüssig dosiert werden kann.

Im erfindungsgemäßen Verfahren wird die Katalysatorkomponente D) typischerweise in Mengen von 0,001 bis 5,0 Gew.-%, bevorzugt 0,01 bis 2,0 Gew.-% und besonders bevorzugt 0,05 bis 1,0 Gew.-% bezogen auf Festgehalt des Verfahrensprodukts eingesetzt.

Als Bestandteile der Komponente E) können im erfindungsgemäßen Verfahren beispielsweise Lösemittel oder Reaktivverdünner eingesetzt werden.

Geeignete Lösemittel sind gegenüber den vorhandenen funktionellen Gruppen des Verfahrensprodukts vom Zeitpunkt der Zugabe bis zum Ende des Verfahrens inert. Geeignet sind z.B. in der Lacktechnik verwendete Lösemittel wie Kohlenwasserstoffe, Ketone und Ester, z.B. Toluol, Xylol, Isooctan, Aceton, Butanon, Methylisobutylketon, Ethylacetat, Butylacetat, Tetrahydrofuran, N-Methylpyrrolidon, Dimethylacetamid, Dimethylformamid, wobei jedoch bevorzugt kein Lösungsmittel zugesetzt wird.

Als Reaktivverdünner können Verbindungen mitverwendet werden, die bei der UV-Härtung ebenfalls (co)polymerisieren und somit in das Polymernetzwerk mit einbauen und gegenüber NCO-Gruppen inert sind. Solche Reaktivverdünner sind in P. K. T. Oldring (Ed.), Chemistry & Technology of UV & EB Formulations For Coatings, Inks & Paints, Vol. 2, 1991, SITA Technology, London, S. 237 - 285 exemplarisch beschrieben. Dies können Ester der Acrylsäure oder Methacrylsäure, bevorzugt der Acrylsäure mit mono- oder mehrfachfunktionellen Alkoholen sein. Als Alkohole eignen sich beispielsweise die isomeren Butanole, Pentanole, Hexanole, Heptanole, Octanole, Nonanole und Decanole, weiterhin cycloaliphatische Alkohole wie Isobornol, Cyclohexanol und alkylierte Cyclohexanole, Dicyclopentanol, arylaliphatischer Alkohole wie Phenoxyethanol und Nonylphenylethanol, sowie Tetrahydrofurfurylalkohole. Weiterhin können alkoxylierte Derivate dieser Alkohole verwendet werden. Geeignete zweiwertige Alkohole sind beispielsweise Alkohole wie Ethylenglykol, Propandiol-1,2, Propandiol-1,3, Diethylenglykol, Dipropylenglykol, die isomeren Butandiole, Neopentylglykol, Hexandiol-1,6, 2-Ethylhexandiol und Tripropylenglykol oder auch alkoxylierte Derivate dieser Alkohole. Bevorzugte zweiwertige Alkohole sind Hexandiol-1,6, Dipropylenglykol und Tripropylenglykol. Geeignete dreiwertige Alkohole sind Glycerin oder Trimethylolpropan oder deren alkoxylierte Derivate. Vierwertige Alkohole sind Pentaerythrit oder dessen alkoxylierte Derivate.

Die erfindungsgemäßen Bindemittel müssen gegen vorzeitige Polymerisation stabilisiert werden. Daher gibt man als Bestandteil von Komponente E) vor und/oder während der Reaktion bevorzugt phenolische Stabilisatoren zu, die die Polymerisation inhibieren. Verwendet werden dabei Phenole wie para-Methoxyphenol, 2,5-Di-tert.-Butylhydrochinon oder 2,6-Di-tert.-butyl-4-methylphenol. Geeignet sind auch N-Oxyl-Verbindungen zur Stabilisierung wie z.B. 2,2,6,6-Tetramethylpiperidin-N-Oxid (TEMPO) oder seine Derivate. Ebenso können die Stabilisatoren auch chemisch mit in das Bindemittel eingebaut werden, dabei eignen sich Verbindungen der oben genannten Klassen insbesondere wenn sie noch weitere freie aliphatische Alkoholgruppen oder primäre oder sekundäre Amingruppen tragen und damit über Urethan- oder Harnstoff-Gruppen chemisch an Verbindungen der Komponente A) gebunden werden können. Hierfür besonders geeignet sind 2,2,6,6-Tetramethyl-4-hydroxy-piperidin-N-Oxid. Bevorzugt sind phenolische Stabilisatoren insbesondere para-Methoxyphenol und/oder 2,6-Di-tert.-butyl-4-methylphenol

Andere Stabilisatoren wie z. B. Verbindungen der Klasse der HALS (HALS = hindered amine light stabilizers) werden dagegen weniger bevorzugt in E) eingesetzt, da sie bekanntermaßen keine so effektive Stabilisierung zu ermöglichen und vielmehr zu einer "schleichenden" radikalischen Polymerisation ungesättigter Gruppen führen können.

Zur Stabilisierung der Reaktionsmischung, insbesondere der ungesättigten Gruppen gegen vorzeitige Polymerisation, kann ein sauerstoffhaltiges Gas, bevorzugt Luft, in und/oder über das Reaktionsgemisch geleitet werden. Es ist bevorzugt, dass das Gas einen möglichst geringen Anteil an Feuchtigkeit besitzt, um unerwünschte Reaktion bei Vorhandensein von Isocyanat zu verhindern.

In der Regel wird während der Herstellung der erfindungsgemäßen Bindemittel ein Stabilisator zugesetzt und abschließend um eine Langzeitstabilität zu erreichen nochmals mit einem phenolischen Stabilisator nachstabilisiert und ggf. mit Luft das Reaktionsprodukt gesättigt.

Im erfindungsgemäßen Verfahren wird die Stabilisatorkomponente typischerweise in Mengen von 0,001 bis 5,0 Gew.-%, bevorzugt 0,01 bis 2,0 Gew.-% und besonders bevorzugt 0,05 bis 1,0 Gew.-% bezogen auf Festgehalt des Verfahrensprodukts eingesetzt.

Das Verhältnis von OH-Gruppen aus der Komponente B) zu der Summe aus NCO- und Uretdiongruppen aus A) beträgt von 1,5 : 1,0 bis 1,0 : 1,9, bevorzugt von 1,0 : 1,0 bis 1,0 : 1,9, besonders bevorzugt von 1,0 : 1,0 bis 1,0 : 1,2.

Das erfindungsgemäße Verfahren wird bevorzugt bei Temperaturen von 20 bis 130°C, besonders bevorzugt von 40 bis 100°C, ganz besonders bevorzugt bei 80 bis 90°C durchgeführt.

Üblicherweise reagieren die ggf. vorhandenen NCO-Gruppen schneller mit den Hydroxylgruppen der Komponente B) als die Uretdiongruppen der Komponente A). Es ist daher möglich, wenn in B) mehrere unterschiedliche Bestandteile vorhanden sind, durch die Zugabereihenfolge der Bestandteile die Urethanisierung und Allophanatisierung entsprechend so zu steuern, dass ein Bestandteil von B) bevorzugt unter Urethanisierung, während der zuletzt zugegebene bevorzugt unter Allophanatisierung eingebaut wird.

Die Umsetzung der Komponente A) mit B) und weiteren gegenüber NCO-Gruppen reaktiven Verbindungen C) ist dann abgeschlossen, sobald alle NCO-Gruppen gemäß der gewählten stöchiometrischen Verhältnisse mit NCO-reaktiven Gruppen aus B) und C) abreagiert haben und alle Uretdiongruppen gemäß der gewählten stöchiometrischen Verhältnisse mit den Hydroxylgruppen aus B abreagiert haben.

Es ist allerdings auch möglich, die Allophanatisierung durch Zugabe von den Katalysator desaktivierenden Verbindungen (z.B. starke Säuren wie saure Phosphorsäureester) oder Zugabe von weiteren isocyanathaltigen Verbindungen, die die verbleibenden Verbindungen der Komponente B) abfangen, zu beenden.

Es ist unerheblich, ob das erfindungsgemäßen Verfahren kontinuierlich z.B. in einem Statik-Mischer, Extruder oder Kneter oder diskontinuierlich z.B. in einem Rührreaktor durchgeführt wird.

Bevorzugt wird das erfindungsgemäße Verfahren in einem Rührreaktor durchgeführt.

Der Verlauf der Reaktion kann durch geeignete im Reaktionsgefäß installierte Meßgeräte und/oder anhand von Analysen an entnommenen Proben verfolgt werden. Geeignete Verfahren sind dem Fachmann bekannt. Es handelt sich beispielsweise um Viskositätsmessungen, Messungen des Brechungsindex, des OH-Gehalts, Gaschromatographie (GC), kernmagnetische Resonanzspektroskopie (NMR), Infrarotspektroskopie (IR) und nahe Nahinfrarotspektroskopie (NIR). Bevorzugt ist die IR-Kontrolle auf ggf. vorhandene freie NCO Gruppen (für aliphatische NCO-Gruppen, Bande bei ca. v = 2272 cm⁻¹) sowie insbesondere aufUretdiongruppen (z.B. Bande für Uretdione auf Basis Hexamethylendiisocyanat bei v = 1761 cm⁻¹) und GC-Untersuchungen auf nicht umgesetzte Verbindungen aus B) und C).

Gemäß der Erfindung wird zunächst A) mit B) bis zur vollständigen Reaktion der NCO-Gruppen umgesetzt. Wobei E) oder Teile davon bereits anwesend sein können. Anschließend wird durch Zugabe von D) und zusätzlich ggf. durch Temperaturanpassung die Reaktion der Uretdiongruppen von A) mit B) gestartet.

In einer besonders bevorzugten Ausführungsform erfolgt die Umsetzung der Isocyanatgruppen und der Uretdiongruppen mit einem Überschuss an Hydroxylgruppen der Komponente B. Die nach der Umsetzung A) mit B) unter Katalyse von D) verbleibenden Hydroxylgruppen werden anschließend bevorzugt mit weiteren isocyanathaltigen Verbindungen, insbesondere mit solchen als mögliche Bestandteile der Komponente B) beschriebenen unter Urethanisierung umgesetzt.

Die nach dem erfindungsgemäßen Verfahren erhältlichen ungesättigten Allophanate weisen bevorzugt Viskositäten gemessen mit einem Kegel-Platte-Viskosimeter bei 23°C von ≤ 100 000 mPas, besonders bevorzugt ≤ 75 000 mPas auf.

Die nach dem erfindungsgemäßen Verfahren erhältlichen ungesättigten Allophanate weisen bevorzugt zahlenmittlere Molekulargewichte Mₙ von 600 bis 3000 g/mol, besonders bevorzugt 750 bis 1500 g/mol auf.

Die nach dem erfindungsgemäßen Verfahren erhältlichen ungesättigten Allophanate weisen Gehalte an freien Di- bzw. Triisocyanatmonomeren von unter 0,5 Gew.-%, bevorzugt unter 0,1 Gew.-% auf.

Die erfindungsgemäßen Bindemittel können zur Herstellung von Beschichtungen und Lacken sowie Klebstoffen, Druckfarben, Gießharzen, Dentalmassen, Schlichten, Photoresisten, Stereolithographiesystemen, Harzen für Verbundwerkstoffe und Dichtungsmassen verwendet werden. Im Falle der Verklebung oder Abdichtung ist allerdings Voraussetzung, dass bei UV-Stahlenhärtung mindestens eines der beiden zu verklebenden oder miteinander abzudichtenden Substrate für UV-Strahlung durchlässig, also i. d. R. transparent sein muss. Bei der Elektronenstrahlung ist auf eine hinreichende Durchlässigkeit für Elektronen zu achten. Bevorzugt ist die Verwendung in Lacken und Beschichtungen.

Ein weiterer Gegenstand der Erfindung sind Beschichtungsmittel enthaltend
a) ein oder mehrere erfindungsgemäß erhältliche Bindemittel
b) gegebenenfalls ein oder mehrere Polyisocyanate mit freien oder blockierten Isocyanatgruppen, die frei sind von unter Einwirkung aktinischer Strahlung mit ethylenisch ungesättigten Verbindungen unter Polymerisation reagierenden Gruppen,
c) gegebenenfalls weitere von denen aus a) verschiedene Verbindungen, die unter Einwirkung aktinischer Strahlung mit ethylenisch ungesättigten Verbindungen unter Polymerisation reagierende Gruppen und gegebenenfalls freie oder blockierte NCO-Gruppen aufweisen,
d) gegebenenfalls ein oder mehrere mit Isocyanaten reagierende, aktiven Wasserstoff enthaltende Verbindungen
e) Initiatoren,
f) gegebenenfalls Lösemittel und
g) gegebenenfalls Hilfs- und Zusatzstoffe.

Die Polyisocyanate der Komponente b) sind dem Fachmann an sich bekannt. Bevorzugt werden hier gegebenenfalls mit Isocyanurat-, Allophanat-, Biuret-, Uretdion- und/oder Iminooxadiazintriongruppen modifizierte Verbindungen auf Hexamethylendiisocyanat-, Isophorondiisocyanat-, 4,4'-Diisocyanatodicyclohexylmethan- und/oder Trimethylhexamethylendiisocyanat-Basis verwendet.

Die NCO-Gruppen können dabei auch blockiert sein, wobei als Blockierungsmittel die bereits bei Beschreibung der Komponente A) genannten Verbindungen zum Einsatz kommen..

Zu den Verbindungen der Komponente c) gehören Verbindungen wie insbesondere Urethanacrylate bevorzugt auf Hexamethylendiisocyanat-, Isophorondiisocyanat-, 4,4'-Diisocyanatodicyclohexylmethan- und/oder Trimethylhexamethylendiisocyanat-Basis, welche gegebenenfalls mit Isocyanurat-, Allophanat-, Biuret-, Uretdion- und/oder Iminooxadiazintriongruppen modifiziert sein können, welche keine gegenüber Isocyanatgruppen reaktive, aktiven Wasserstoff enthaltene Funktionen aufweisen.

NCO-haltige Urethanacrylate sind kommerziell bei Bayer MaterialScience AG, Leverkusen, DE als Roskydal^{®} UA VP LS 2337, Roskydal^{®} UA VP LS 2396 oder Roskydal^{®} UA XP 2510 erhältlich.

Weiterhin können die bereits beschriebenen und in der Technik der strahlenhärtenden Beschichtungen bekannten Reaktivverdünner als Bestandteil von c) verwendet werden, sofern sie keine mit NCO-Gruppen reaktiven Gruppen enthalten.

Verbindungen der Komponente d) können gesättigt oder ungesättigt sein. Mit NCO-Gruppen reagierende chemische Funktionalitäten sind aktivierte Wasserstoffatome enthaltende Funktionalitäten wie Hydroxyl, Amin oder Thiol.

Bevorzugt sind gesättigte Polyhydroxyverbindungen , z.B. die an sich aus der Technolgie des Beschichtens, des Klebens, der Druckfarben oder der Dichtungsmassen bekannten Polyetherpolyole, Polyesterpolyole, Polycarbonatpolyole, Poly(meth)acrylatpolyole, Polyurethanpolyole, die keine unter Einwirkung aktinischer Strahlung mit ethylenisch ungesättigten Verbindungen unter Polymerisation reagierende Gruppen aufweisen.

Ungesättigte hydroxyfunktionelle Verbindungen sind z.B. die in der Technik der strahlenhärtenden Beschichtungen bekannten Epoxyacrylate, Polyesteracrylate, Polyetheracrylate, Urethanacrylate sowie acrylierte Polyacrylate, die eine OH-Zahl von 30 bis 300 mg KOH/g aufweisen.

Weiterhin können die bereits beschriebenen und in der Technik der strahlenhärtenden Beschichtungen bekannten Reaktivverdünner als Bestandteil von d) verwendet werden, sofern sie mit NCO-Gruppen reaktive Gruppen enthalten.

Als Initiatoren der Komponente e) für eine radikalische Polymerisation können durch Strahlung und/oder thermisch aktivierbare Initiatoren zum Einsatz kommen. Fotoinitiatoren, die durch UV-oder sichtbares Licht aktiviert werden, sind hierbei bevorzugt. Fotoinitiatoren sind an sich bekannte, kommerziell vertriebene Verbindungen, wobei zwischen unimolekularen (Typ I) und bimolekularen (Typ II) Initiatoren unterschieden wird. Geeignete (Typ I)-Systeme sind aromatische Ketonverbindungen, z.B. Benzophenone in Kombination mit tertiären Aminen, Alkylbenzophenone, 4,4'-Bis(dimethylamino)benzophenon (Michlers Keton), Anthron und halogenierte Benzophenone oder Mischungen der genannten Typen. Weiter geeignet sind (Typ II)-Initiatoren wie Benzoin und seine Derivate, Benzilketale, Acylphosphinoxide z.B. 2,4,6-Trimethyl-benzoyl-diphenylphosphinoxid, Bisacylphosphinoxide, Phenylglyoxylsäureester, Campherchinon, α-Aminoalkylphenone, α,α-Dialkoxyacetophenone und α-Hydroxyalkylphenone.

Die Initiatoren, die in Mengen zwischen 0,1 und 10 Gew.-%, vorzugsweise 0,1 bis 5 Gew.-%, bezogen auf das Gewicht des Lackbindemittels, eingesetzt werden, können als einzelne Substanz oder, wegen häufiger vorteilhafter synergistischer Effekte, auch in Kombination miteinander verwendet werden.

Werden Elektronenstrahlen statt UV-Strahlung verwendet, so benötigt man keinen Photoinitiator. Elektronenstrahlung wird wie dem Fachmann bekannt ist, mittels thermischer Emission erzeugt und über eine Potentialdifferenz beschleunigt. Die energiereichen Elektronen schlagen dann durch eine Titanfolie und werden auf die zu härtenden Bindemittel gelenkt. Die allgemeinen Prinzipien der Elektronenstrahlhärtung sind in "Chemistry & Technology of UV & EB Formulations for Coatings, Inks & Paints", Vol. 1, P K T Oldring (Ed.), SITA Technology, London, England, S. 101-157, 1991 im Detail beschrieben.

Im Falle einer thermischen Härtung der aktivierten Doppelbindungen, kann diese auch unter Zusatz von thermisch zerfallenden Radikalbildnern erfolgen. Geeignet sind, wie dem Fachmann bekannt ist, z.B. Peroxyverbindungen wie Dialkoxydicarbonate wie z.B. Bis(4-tert-butylcyclohexyl)-peroxydicarbonat, Dialkylperoxide wie z.B. Dilaurylperoxid, Perester aromatischer oder aliphatischer Säuren wie z.B. tert.-Butylperbenzoat oder tert.-Amylperoxy 2-ethylhexanoat, anorganische Peroxide wie z. B. Ammoniumperoxodisulfat, Kaliumperoxodisulfat, organische Peroxide wie z.B. 2,2-Bis(tert.-butylperoxy)butan, Dicumylperoxid, tert.-Butylhydroperoxid oder auch Azoverbindungen wie 2,2'-Azobis[N-(2-propenyl)-2-methylpropionamide], 1-[(cyano-1-methylethyl)azo]formamide, 2,2'-Azobis(N-butyl-2-methylpropionamide), 2,2'-Azobis(N-cyclohexyl-2-methylpropionamide), 2,2'-Azobis {2-methyl-N-[2-(1-hydroxybutyl)]propionamide}, 2,2'-Azobis {2methyl-N-[2-(1-hydroxybutyl)]propionamide, 2,2'-Azobis{2-methyl-N-[1,1-bis(hydroxymethyl)-2-hydroxyethyl] propionamide. Möglich sind auch hochsubstituierte 1,2-Diphenylethane (Benzpinakole), wie z. B. 3,4-Dimethyl-3,4-diphenylhexan, 1,1,2,2-Tetraphenyl-ethandiol-1,2 oder auch deren silylierten Derivate.

Es ist auch möglich eine Kombination von durch UV-Licht und thermisch aktivierbaren Initiatoren zu verwenden.

Zu den Hilfs- und Zusatzstoffen der Komponente e) gehören Lösemittel der vorstehend unter E) genannten Art.

Des Weiteren können in e) zur Erhöhung der Wetterstabilität der gehärteten Lackschicht auch UV-Absorber und/oder HALS-Stabilisatoren enthalten sein. Bevorzugt ist die Kombination. Erstere sollten einen Absorptionsbereich von maximal 390 nm haben wie Triphenyltriazintypen (z.B. Tinuvin^{®} 400 (Ciba Spezialitätenchemie GmbH, Lampertheim, DE)), Benztriazole wie Tinuvin^{®} 622 (Ciba Spezialitätenchemie GmbH, Lampertheim, DE) oder Oxalsäuredianilide (z. B. Sanduvor^{®} 3206 (Clariant, Muttenz, CH) )) und werden in 0,5 - 3,5 Gew.-% bezogen auf Festharz zugegeben. Geeignete HALS-Stabilisatoren sind kommerziell erhältlich (Tinuvin^{®} 292 oder Tinuvin^{®} 123 (Ciba Spezialitätenchemie GmbH, Lampertheim, DE) oder Sanduvor^{®} 3258 (Clariant, Muttenz, CH). Bevorzugte Mengen sind 0,5-2,5 Gew.-% bezogen auf Festharz.

Ebenfalls können in e) Pigmente, Farbstoffe, Füllstoffe, Verlaufs- und Entlüftungsadditive enthalten sein.

Darüber hinaus können, sofern erforderlich die aus der Polyurethanchemie bekannten Katalysatoren zur Beschleunigung der NCO/OH-Reaktion in e) enthalten sein. Dies sind z.B. Zinn- oder Zinksalze oder Zinnorganoverbindungen, Zinn- und/oder Zinkseifen wie z.B. Zinnoctoat, Dibutylzinndilaurat, Dibutylzinnoxid oder tertiäre Amine wie z. B. Diazabicyclo[2,2,2]octan (DABCO).

Das Auftragen der erfindungsgemäßen Beschichtungsmittel auf das zu beschichtende Material erfolgt mit den in der Beschichtungstechnologie üblichen und bekannten Methoden wie Spritzen, Rakeln, Walzen, Gießen, Tauchen, Schleudern, Streichen oder Sprühen oder durch Druck-Techniken wie Sieb-, Tief-, Flexo- oder Offsetdruck sowie durch Transfer-Methoden.

Geeignete Substrate sind beispielsweise Holz, Metall, insbesondere auch Metall wie es in den Anwendungen der sogenannten Draht-, Coil-, Can- oder Container-Lackierung verwendet wird, weiterhin Kunststoff auch in Form von Folien, insbesondere ABS, AMMA, ASA, CA, CAB, EP, UF, CF, MF, MPF, PF, PAN, PA, PE, HDPE, LDPE, LLDPE, UHMWPE, PET, PMMA, PP, PS, SB, PUR, PVC, RF, SAN, PBT, PPE, POM, PUR-RIM, SMC, BMC, PP-EPDM, und UP (Kurzbezeichnungen nach DIN 7728T1), Papier, Leder, Textilien, Filz, Glas, Holz, Holzwerkstoffe, Kork, anorganisch gebundene Substrate wie Holz- und Faserzementplatten, elektronische Baugruppen oder mineralische Untergründe. Es können auch Substrate, die aus verschiedenen der vorgenannten Materialien bestehen, oder bereits beschichtete Substrate wie Fahrzeuge, Flugzeuge oder Schiffe sowie deren Teile, insbesondere Karosserien oder Anbauteile lackiert werden. Es ist auch möglich die Beschichtungsmittel nur temporär auf ein Substrat aufzubringen, dann teilweise oder vollständig zu härten und gegebenenfalls wieder abzulösen, um z.B. Folien herzustellen.

Zur Aushärtung können durch Ablüften z.B. enthaltene Lösemittel ganz oder teilweise entfernt werden.

Anschließend oder gleichzeitig können der oder die ggf. notwendigen thermischen und der und die photochemischen Aushärtungsprozesse nacheinander oder gleichzeitig durchgeführt werden.

Falls notwendig kann die thermische Härtung kann bei Raumtemperatur aber auch bei erhöhter Temperatur, vorzugsweise bei 40 bis 160°C, bevorzugte bei 60 bis 130°C, besonders bevorzugte bei 80 bis 110°C erfolgen.

Bei Verwendung von Photoinitiatoren in d) erfolgt die Strahlungshärtung bevorzugt durch Einwirkung energiereicher Strahlung, also UV-Strahlung oder Tageslicht, z.B. Licht der Wellenlänge 200 bis 700 nm oder durch Bestrahlen mit energiereichen Elektronen (Elektronenstrahlung, 150 bis 300 keV). Als Strahlungsquellen für Licht oder UV-Licht dienen beispielsweise Hoch- oder Mitteldruckquecksilberdampflampen, wobei der Quecksilberdampf durch Dotierung mit anderen Elementen wie Gallium oder Eisen modifiziert sein kann. Laser, gepulste Lampen (unter der Bezeichnung UV-Blitzlichtstrahler bekannt), Halogenlampen oder Eximerstrahler sind ebenfalls möglich. Die Strahler können bauartbedingt oder durch Einsatz spezieller Filter und/oder Reflektoren so ausgestattet sein, das der Austritt eines Teils des UV-Spektrums verhindert wird. Beispielsweise kann z.B. aus arbeitshygienischen Gründen die dem UV-C oder UV-C und UV-B zugeordnete Strahlung herausgefiltert werden. Die Strahler können ortsunbeweglich installiert sein, so dass das zu bestrahlende Gut mittels einer mechanischen Vorrichtung an der Strahlungsquelle vorbeibewegt wird oder die Strahler können beweglich sein und das zu bestrahlende Gut verändert bei der Härtung seinen Ort nicht. Die üblicherweise zur Vernetzung ausreichende Strahlungsdosis bei UV-Härtung liegt im Bereich von 80 bis 5000 mJ/cm².

Die Bestrahlung kann gegebenenfalls auch unter Ausschluss von Sauerstoff, z. B. unter InertgasAtmosphäre oder Sauerstoff-reduzierter Atmosphäre durchgeführt werden. Als Inertgase eignen sich bevorzugt Stickstoff, Kohlendioxid, Edelgase oder Verbrennungsgase. Des Weiteren kann die Bestrahlung erfolgen, indem die Beschichtung mit für die Strahlung transparenten Medien abgedeckt wird. Beispiele hierfür sind z.B. Kunststofffolien, Glas oder Flüssigkeiten wie Wasser.

Je nach Strahlungsdosis und Aushärtungsbedingungen sind Typ und Konzentration des gegebenenfalls verwendeten Initiators in dem Fachmann bekannter Weise zu variieren.

Besonders bevorzugt werden zur Härtung Quecksilberhochdruckstrahler in ortsfesten Anlagen eingesetzt. Fotoinitiatoren werden dann in Konzentrationen von 0,1 bis 10 Gew.-%, besonders bevorzugt 0,2 bis 3,0 Gew.-% bezogen auf den Festkörper der Beschichtung eingesetzt. Zur Härtung dieser Beschichtungen wird bevorzugt eine Dosis von 200 bis 3000 mJ/cm² gemessen im Wellenlängebereich von 200 bis 600 nm verwendet.

Bei Verwendung von thermisch aktivierbaren Initiatoren in d) durch Erhöhung der Temperatur. Die thermische Energie kann dabei durch Strahlung, Wärmeleitung und/oder Konvektion in die Beschichtung eingebracht werden, wobei üblicherweise die in der Beschichtungstechnologie gebräuchlichen Infrarot-Strahler, Nahinfrarotstrahler und/oder Öfen zum Einsatz kommen.

Die applizierten Schichtdicken (vor der Härtung) liegen typischerweise zwischen 0,5 und 5000 µm, bevorzugt zwischen 5 und 1000 µm, besonders bevorzugt zwischen 15 und 200 µm. Bei Verwendung von Lösungsmitteln wird dieses nach der Applikation und vor der Härtung durch die gängigen Methoden entfernt.

### Beispiele

Alle Prozentangaben beziehen sich sofern nicht abweichend angegeben auf Gewichtsprozent.

Die Bestimmung der NCO-Gehalte in % wurde über Rücktitration mit 0,1 mol/l Salzsäure nach Reaktion mit Butylamin vorgenommen, Grundlage DIN EN ISO 11909.

Die Viskositätsmessungen wurden mit einem Kegelplatte Viskosimeter (SM-KP), Viskolab LC3/ISO der Fa. Paar Physica, Ostfildern, DE nach ISO/DIS 3219:1990 durchgeführt.

Infrarot-Spektroskopie wurde an zwischen Natriumchloridplatten aufgetragenen Flüssigkeitsfilmen an einem Gerät Modell 157 von Perkin Elmer, Überlingen, DE.

Gehalt an Restmonomeren bzw. flüchtiger Aufbaukomponenten wurde mittels GC (Methode mit Tetradekan als internem Standard, Ofentemperatur 110°C, Injektortemperatur 150°C, Trägergas Helium, Gerät: 6890 N, Agilent, Waldbronn, DE, Säule: Restek RT 50, 30 m, 0,32 mm Innendurchmesser, Filmdicke 0,25 µm) analysiert.

Die Bestimmung des Festkörpers erfolgte nach DIN 53216/1 Entwurf 4/89, ISO 3251

Die zur Zeit der Versuchsdurchführung herrschende Umgebungstemperatur von 23°C wird als RT bezeichnet.

Desmodur^{®} N 3400: HDI-Polyisocyanat vorwiegend enthaltend Uretdionstruktur, Viskosität 185 mPas/23°C, NCO-Gehalt 21,4 %, Handelsprodukt der Bayer AG, Leverkusen, DE

Desmorapid^{®} Z: Dibutylzinndilaurat (DBTL), Handelsprodukt der Bayer AG, Leverkusen, DE

Darocur^{®} 1173: Photoinitiator, Handelsprodukt der Ciba Spezialitätenchemie GmbH, Lampertheim, DE

Tone^{®} M100: Umsetzungsprodukt aus 2 Äquivalenten ε-Caprolacton mit 1 Äquivalent 2-Hydroxyethylacrylat, OH-Gehalt = 4,97 %, Viskosität = 82 mPas/23°C, Handelsprodukt der Dow, Schwalbach, DE.

Beispiel 1 beschreibt die Herstellung eines geeigneten katalytisch aktiven Carboxylats, das im Beispiel 2 für die Reaktion von uretdiongruppenhaltigen Verbindungen mit ethylenisch ungesättigten Hydroxylverbindungen zu entsprechenden allophanathaltigen Verbindungen eingesetzt wird.

### Beispiel 1 Cholin-ethylhexanoat

In einem Glaskolben mit Rückflußkühler, beheizbarem Ölbad, mechanischem Rührer und Innenthermometer wurden bei RT 272,13 g einer 40-%igen Lösung von Cholinhydroxid und 145,73 g 2-Ethylhexansäure 30 min intensiv gerührt. Am Rotationsverdampfer wurden Wasser und Methanol unter nach und nach auf 20 mbar verstärktem Vakuum bei 30-45°C abdestilliert. Das Produkt wurde dann mit n-Hexan aufgenommen und erneut am Rotationsverdampfer eingeengt und bei 0,1 mbar und 40°C 2 h getrocknet. Man erhält eine leicht gefärbte, viskose Flüssigkeit, deren 1H-NMR-Spektrum equimolare Verhältnisse von Cholin und Ethylhexanoat, aber nur ein schwaches Signal im Bereich aliphatischer Carbonsäuren aufweist

### Beispiel 2 Erfindungsgemäßes allophanathaltiges Bindemittel

In einem Dreihalskolben mit Rückflusskühler, Rührer, Tropftrichter und Luftdurchleitung (0,5 1/h) wurden 263,47 g Desmodur^{®} N3400, 0,50 g 2,6-Di-tert.-butyl-4-methylphenol und 0,07 g Desmorapid^{®} Z bei RT vorgelegt und dann auf 60°C erhitzt. 219,54 g 2-Hydroxyethylacrylat wurden langsam zugetropft, wobei eine maximale Temperatur von 70°C erreicht wurde. Danach wurde das Reaktionsgemisch bei 65 °C gehalten, bis der NCO-Gehalt < 0,1 %. Anschließend wurde eine Mischung aus 14,43 g 2-Hydroxyethylacrylat und 1,49 g des Katalysators aus Beispiel 1 zugetropft. Das Reaktionsgemisch wurde weiter aufgeheizt und bei 80°C gehalten, bis im IR-Spektrum bei v = 1768 cm⁻¹ nach 2,5 h nur ein sehr schwaches Signal für Uretdiongruppen nachweisbar war. Es wurden 0,50 g Isophthalsäuredichlorid zugegeben und rasch auf RT abgekühlt. Gaschromatographisch wurde an einer entnommenen Probe ein Gehalt an Hydroxyethylacrylat von 4,68 % bestimmt. Es erfolgte die Zugabe von 39,0 g Desmodur N3400 und 0,07 g Desmorapid^{®} Z. Es wurde bei 60°C gerührt, bis im IR-Spektrum bei v = 2272 cm⁻¹ kein Signal für die Isocyanatgruppe mehr vorhanden war. Gaschromatographisch wurde an einer entnommenen Probe der Gehalt an Hydroxyethylacrylat mit 0,18 % bestimmt Es wurde ein Produkt mit der Viskosität 64.500 mPas/23°C, einer Farbzahl 104 APHA und einem NCO-Gehalt unter 0,1 % erhalten

### Vergleichsbeispiel 3 Versuch der Herstellung eines allophanathaltigen Bindemittels

Die in US-A 2003 301 537 13 für die Vernetzung von Pulverlacken aus Uretdiongruppen-haltigen Härtern und polymeren Hydroxylverbindungen ohne aktivierte Doppelbindungen beschriebenen Katalysatoren wurden auf Eignung überprüft:

Beispiel 2 wurde wiederholt mit dem Unterschied, dass jetzt 0,51 g Tetrabutylammoniumhydroxid als Katalysator verwendet wurden. Das Reaktionsgemisch wurde aufgeheizt und bei 80°C gehalten, bis im IR-Spektrum bei v = 1768 cm⁻¹ nach 2 h nur ein sehr schwaches Signal für Uretdiongruppen nachweisbar war. Es wurden 0,10 g Benzoylchlorid zugegeben und rasch auf RT abgekühlt. Die Reaktionsmischung trübte dabei ein. Gaschromatographisch wurde an einer entnommenen Probe der Gehalt an Hydroxyethylacrylat mit 2,4 % bestimmt. Zur Reaktionsmischung wurden 5,20 g Desmodur^{®} N3400 gegeben und bei 70°C gerührt, bis im IR-Spektrum bei ν = 2272 cm⁻¹ kein Signal für die Isocyanatgruppe mehr vorhanden war. Gaschromatographisch wurde an einer entnommenen Probe der Gehalt an Hydroxyethylacrylat mit 0,17 % bestimmt Es wurde ein trübes Produkt mit einer Viskosität von 84.000 mPas/23°C und einem NCO-Gehalt von 0 % erhalten.

### Vergleichsbeispiel 4 Versuch der Herstellung eines allophanathaltigen Bindemittels

Die in US-A 2003 301 537 13 für die Vernetzung von Pulverlacken aus Uretdiongruppen-haltigen Härtern und polymeren Hydroxylverbindungen ohne aktivierte Doppelbindungen beschriebenen Katalysatoren wurden auf Eignung überprüft:

Beispiel 2 wurde wiederholt mit dem Unterschied, dass statt des Katalysators aus Beispiel 3 jetzt 0,67 g Tetrabutylammoniumfluorid als Katalysator verwendet wurden. Das Reaktionsgemisch wurde aufgeheizt und bei 80°C gehalten, bis im IR-Spektrum bei v = 1768 cm⁻¹ nach 3 h nur ein sehr schwaches Signal für Uretdiongruppen nachweisbar war. Es wurden 0,10 g Benzoylchlorid zugegeben und rasch auf RT abgekühlt. Die Reaktionsmischung trübte dabei stark ein, es bildete sich ein farbloser Niederschlag. Gaschromatographisch wurde an einer entnommenen Probe der Gehalt an Hydroxyethylacrylat mit 1,7 % bestimmt. Zur Reaktionsmischung wurden 4,30 g Desmodur^{®} N3400 gegeben und bei 70°C gerührt, bis im IR-Spektrum bei v = 2272 cm⁻¹ kein Signal für die Isocyanatgruppe mehr vorhanden war. Gaschromatographisch wurde an einer entnommenen Probe der Gehalt an Hydroxyethylacrylat mit 0,15 % bestimmt Es wurde ein trübes Produkt mit einer Viskosität von 92.000 mPas/23°C und einem NCO-Gehalt von 0 % erhalten.

Die Vergleichsbeispiele 3 und 4 zeigen, dass die für die Vernetzung von Pulverlacken bestehend aus Uretdiongruppen-haltigen Härtern und polymeren Hydroxylverbindungen geeignete Substanzen für eine gezielte Synthese von Allophanaten aus Uretdionen und Alkohlen nicht geeignet sind. Die so erhaltenen Produkte sind getrübt und relativ hochviskos, so dass sie sich zur Herstellung von Beschichtungen nicht eignen.

### Beispiel 5 Lackformulierung und Lack

Ein Teil des Produkts aus Beispiel 2 wurde mit 3,0 % des Photoinitiators Darocur^{®} 1173 intensiv gemischt. Mittels eines Knochen-Rakels mit einem Spalt von 90 µm wurde die Mischung als dünner Film auf eine Glasplatte aufgezogen. Nach UV-Bestrahlung (Quecksilbermitteldruckstrahler, IST Metz GmbH, Nürtingen, DE, 750 mJ/cm²) wurde ein transparente, harte und lösemittelfeste Beschichtung mit einer Pendelhärte 152 s erhalten, die sich durch Stahlwolle (Typ 0/0/0) mit einer auf den Film gerichteten Kraft von 500 g in zehn Doppelhüben kaum verkratzen ließ und sich durch 100 Doppelhübe mit einem Butylacetat getränkten Wattebausch nicht sichtbar veränderte.

## Patentansprüche

1. Verfahren zur Herstellung strahlenhärtender, allophanatgruppenhaltiger Bindemittel, bei dem bei Temperaturen ≤ 130°C
A) eine oder mehrere uretdiongruppenhaltige Verbindungen mit
B) einer oder mehreren OH-funktionellen Verbindungen, die unter Einwirkung aktinischer Strahlung mit ethylenisch ungesättigten Verbindungen unter Polymerisation reagierende Gruppen (strahlenhärtende Gruppen) aufweisen und
C) gegebenenfalls weiteren gegenüber NCO-Gruppen reaktiven Verbindungen in Anwesenheit
D) einer oder mehrerer Verbindungen enthaltend mindestens ein tetrasubstituertes Ammonium- oder Phosphoniumsalz einer aliphatischen oder cycloaliphatischen Carbonsäure als Katalysator und
E) eines oder mehreren Stabilisatoren und optional Hilfs- und Zusatzstoffen
unter Öffnung des Uretdionringes zu Allophanatgruppen umgesetzt werden, wobei das Verhältnis der OH-Gruppen aus der Komponente B) zu der Summe aus NCO- und Uretdiongruppen aus A) von 1,5 : 1,0 bis 1,0 : 1,9 beträgt; zunächst A) mit B) bis zur vollständigen Reaktion der NCO-Gruppen umgesetzt wird - wobei E) oder Teile davon bereits anwesend sein können - um anschließend durch Zugabe von D) und zusätzlich gegebenenfalls durch Temperaturanpassung die Reaktion der Uretdiongruppen von A) mit B) zu starten; die erhältlichen ungesättigten Allophanate Gehalte an freien Di- bzw. Triisocyanatmonomeren von unter 0,5 Gew.-%, bevorzugt unter 0,1 Gew.-% aufweisen; und die Umsetzung der Komponente A) mit B) und weiteren gegenüber NCO-Gruppen reaktiven Verbindungen C) dann abgeschlossen ist, sobald alle NCO-Gruppen gemäß der gewählten stöchiometrischen Verhältnisse mit NCO-reaktiven Gruppen aus B) und C) abreagiert haben und alle Uretdiongruppen gemäß der gewählten stöchiometrischen Verhältnisse mit den Hydroxylgruppen aus B) abreagiert haben.

2. Verfahren zur Herstellung strahlenhärtender, allophanatgruppenhaltiger Bindemittel gemäß Anspruch 1, **dadurch gekennzeichnet, dass** die uretdiongruppenhaltigen Verbindungen der Komponente A) auf Hexamethylendiisocyanat basieren.

3. Verfahren zur Herstellung strahlenhärtender, allophanatgruppenhaltiger Bindemittel gemäß Anspruch 1 oder 2, **dadurch gekennzeichnet, dass** in Komponente B) 2-Hydroxyethylacrylat und/oder 4-Hydroxybutylacrylat eingesetzt werden.

4. Verfahren zur Herstellung strahlenhärtender, allophanatgruppenhaltiger Bindemittel gemäß einem der Ansprüche 1 bis 3, **dadurch gekennzeichnet, dass** in Komponente D) ausschließlich tetrasubstituerte Ammonium- oder Phosphoniumsalze einer aliphatischen oder cycloaliphatischen Carbonsäure als Katalysator eingesetzt werden.

5. Verfahren zur Herstellung strahlenhärtender, allophanatgruppenhaltiger Bindemittel gemäß einem der Ansprüche 1 bis 4, **dadurch gekennzeichnet, dass** in Komponente D) als Katalysator Tetrabutylammonium-2-ethylhexanoat, Tetrabutylammonium-pivalat, Cholin-2-ethylhexanoat, Cholin-pivalat, Methylcholin-2-ethylhexanoat und/oder Methylcholin-pivalat verwendet wird.

6. Verfahren zur Herstellung strahlenhärtender, allophanatgruppenhaltiger Bindemittel gemäß einem der Ansprüche 1 bis 5, **dadurch gekennzeichnet, dass** die Verfahrenstemperaturen 40 bis 100°C betragen.

7. Strahlenhärtende, allophanatgruppenhaltige Bindemittel, die nach einem Verfahren gemäß einem der Ansprüche 1 bis 6 erhältlich sind.

8. Verwendung der strahlenhärtenden, allophanatgruppenhaltigen Bindemittel nach Anspruch 7 bei der Herstellung von Beschichtungen, Lacken, Klebstoffen, Druckfarben, Gießharzen, Dentalmassen, Schlichten, Photoresisten, Stereolithographiesystemen, Harzen für Verbundwerkstoffe und Dichtungsmassen.

9. Beschichtungsmittel enthaltend
a) ein oder mehrere strahlenhärtende, allophanatgruppenhaltige Bindemittel gemäß Anspruch 7,
b) gegebenenfalls ein oder mehrere Polyisocyanate mit freien oder blockierten Isocyanatgruppen, die frei sind von unter Einwirkung aktinischer Strahlung mit ethylenisch ungesättigten Verbindungen unter Polymerisation reagierenden Gruppen,
c) gegebenenfalls weitere von denen aus a) verschiedene Verbindungen, die unter Einwirkung aktinischer Strahlung mit ethylenisch ungesättigten Verbindungen unter Polymerisation reagierende Gruppen und gegebenenfalls freie oder blockierte NCO-Gruppen aufweisen,
d) gegebenenfalls ein oder mehrere mit Isocyanaten reagierende, aktiven Wasserstoff enthaltende Verbindungen
e) Initiatoren,
f) gegebenenfalls Lösemittel und
g) gegebenenfalls Hilfs- und Zusatzstoffe.

10. Substrate beschichtet mit Beschichtungen erhältlich aus strahlenhärtenden, allophanatgruppenhaltigen Bindemitteln gemäß Anspruch 7.

## Claims

1. Process for preparing radiation-curing binders containing allophanate groups, where, at temperatures ≤ 130°C
A) one or more compounds containing uretdione groups is or are reacted with
B) one or more OH-functional compounds which contain groups which react, with polymerization, with ethylenically unsaturated compounds on exposure to actinic radiation (radiation-curing groups), and
C) optionally further NCO-reactive compounds in the presence
D) of one or more compounds containing at least one tetrasubstituted ammonium or phosphonium salt of an aliphatic or cycloaliphatic carboxylic acid as catalyst, and
E) one or more stabilizers and optionally auxiliaries and additives
to form allophanate groups by opening the uretdione ring, wherein the ratio of the OH groups from component B) to the sum of NCO and uretdione groups from A) is from 1.5:1.0 to 1.0:1.9; first A) is reacted with B) until reaction of the NCO groups is complete - at this stage, E) or parts thereof may already be present - and then the reaction of the uretdione groups of A) with B) is initiated by addition of D) and additionally, optionally, by temperature adaptation; the unsaturated allophanates obtainable contain amounts of free di- and/or triisocyanate monomers of below 0.5% by weight, preferably below 0.1% by weight; and the reaction of component A) with B) and further NCO-reactive compounds C) is over as soon as all of the NCO groups, in accordance with the chosen stoichiometric proportions, have reacted with NCO-reactive groups from B) and C) and all uretdione groups, in accordance with the chosen stoichiometric proportions, have reacted with the hydroxyl groups from B).

2. Process for preparing radiation-curing binders containing allophanate groups according to Claim 1, **characterized in that** the compounds of component A) containing uretdione groups are based on hexamethylene diisocyanate.

3. Process for preparing radiation-curing binders containing allophanate groups according to Claim 1 or 2, **characterized in that** in component B) 2-hydroxyethyl acrylate and/or 4-hydroxybutyl acrylate are used.

4. Process for preparing radiation-curing binders containing allophanate groups according to Claim 1 to 3, **characterized in that** in component D) only tetrasubstituted ammonium or phosphonium salts of an aliphatic or cycloaliphatic carboxylic acid are used as catalyst.

5. Process for preparing radiation-curing binders containing allophanate groups according to any one of Claims 1 to 4, **characterized in that** in component D) as catalyst tetrabutylammonium 2-ethylhexanoate, tetrabutylammonium pivalate, choline 2-ethylhexanoate, choline pivalate, methylcholine 2-ethylhexanoate and/or methylcholine pivalate is used.

6. Process for preparing radiation-curing binders containing allophanate groups according to any one of Claims 1 to 5, **characterized in that** the process temperatures are 40 to 100°C.

7. Radiation-curing binders containing allophanate groups, obtainable by a process according to any one of Claims 1 to 6.

8. Use of the radiation-curing binders containing allophanate groups according to Claim 7 in the preparation of coatings, coating materials, adhesives, printing inks, casting resins, dental compounds, sizes, photoresists, stereolithography systems, resins for composite materials and sealants.

9. Coating compositions comprising
a) one or more radiation-curing binders containing allophanate groups, according to Claim 7,
b) optionally one or more polyisocyanates containing free or blocked isocyanate groups, which are free from groups which react, with polymerization, with ethylenically unsaturated compounds on exposure to actinic radiation,
c) optionally other compounds, different from those of a), which contain groups which react, with polymerization, with ethylenically unsaturated compounds on exposure to actinic radiation, and optionally contain free or blocked NCO groups,
d) optionally one or more isocyanate-reactive compounds containing active hydrogen,
e) initiators,
f) optionally solvents and
g) optionally auxiliaries and additives.

10. Substrates coated with coatings obtainable from radiation-curing binders containing allophanate groups, according to Claim 7.

## Revendications

1. Procédé pour la préparation de liants contenant des groupes allophanate, durcissant sous l'effet d'un rayonnement, dans lequel on transforme, à des températures ≤ 130°C,
A) un ou plusieurs composés contenant des groupes uretdione avec
B) un ou plusieurs composés à fonctionnalité OH, qui présentent des groupes réagissant avec polymérisation sous l'effet d'un rayonnement actinique avec des composés éthyléniquement insaturés (groupes durcissant sous l'effet d'un rayonnement) et
C) le cas échéant d'autres composés réactifs par rapport aux groupes NCO, en présence
D) d'un ou de plusieurs composés contenant au moins un sel d'ammonium ou de phosphonium tétrasubstitué d'un acide carboxylique aliphatique ou cycloaliphatique comme catalyseur et
E) d'un ou de plusieurs stabilisants et éventuellement d'adjuvants et d'additifs
avec ouverture du cycle uretdione en groupes allophanate, le rapport des groupes OH du composant B) à la somme de groupes NCO et uretdione de A) valant de 1,5:1,0 à 1,0:1,9 ; A) étant d'abord transformé avec B) jusqu'à la réaction complète des groupes NCO - E) ou des parties de celui-ci pouvant déjà être présent(es) - pour ensuite démarrer, par addition de D) et en plus, le cas échéant, par adaptation de la température, la réaction des groupes uretdione de A) avec B) ; les allophanates insaturés pouvant être obtenus présentant des teneurs en monomères de type diisocyanate ou triisocyanate libre inférieures à 0,5% en poids, de préférence inférieures à 0,1% en poids ; et la transformation du composant A) avec B) et d'autres composés C) réactifs par rapport aux groupes NCO étant terminée dès que tous les groupes NCO ont réagi avec les groupes réactifs par rapport aux groupes NCO de B) et C), selon les rapports stoechiométriques choisis et que tous les groupes uretdione ont réagi avec les groupes hydroxyle de B) selon les rapports stoechiométriques choisis.

2. Procédé pour la préparation de liants contenant des groupes allophanate, durcissant sous l'effet d'un rayonnement selon la revendication 1, **caractérisé en ce que** les composés contenant des groupes uretdione du composant A) sont à base de diisocyanate d'hexaméthylène.

3. Procédé pour la préparation de liants contenant des groupes allophanate, durcissant sous l'effet d'un rayonnement selon la revendication 1 ou 2, **caractérisé en ce qu'**on utilise, dans le composant B), de l'acrylate de 2-hydroxyéthyle et/ou de l'acrylate de 4-hydroxypropyle.

4. Procédé pour la préparation de liants contenant des groupes allophanate, durcissant sous l'effet d'un rayonnement, selon l'une quelconque des revendications 1 à 3, **caractérisé en ce qu'**on utilise, dans le composant D), exclusivement des sels d'ammonium ou de phosphonium tétrasubstitués d'un acide carboxylique aliphatique ou cycloaliphatique comme catalyseur.

5. Procédé pour la préparation de liants contenant des groupes allophanate, durcissant sous l'effet d'un rayonnement, selon l'une quelconque des revendications 1 à 4, **caractérisé en ce qu'**on utilise, dans le composant D), comme catalyseur, du 2-éthylhexanoate de tétrabutylammonium, du pivalate de tétrabutylammonium, du 2-éthylhexanoate de choline, du pivalate de choline, du 2-éthylhexanoate de méthylcholine et/ou du pivalate de méthylcholine.

6. Procédé pour la préparation de liants contenant des groupes allophanate, durcissant sous l'effet d'un rayonnement, selon l'une quelconque des revendications 1 à 5, **caractérisé en ce que** les températures de procédé sont de 40 à 100°C.

7. Liants contenant des groupes allophanate, durcissant sous l'effet d'un rayonnement, pouvant être obtenus selon un procédé selon l'une quelconque des revendications 1 à 6.

8. Utilisation des liants contenant des groupes allophanate, durcissant sous l'effet d'un rayonnement selon la revendication 7 lors de la préparation de revêtements, de laques, d'adhésifs, d'encres d'imprimerie, de résines coulées, de masses dentaires, de barbotines, de résines photosensibles, de systèmes de stéréolithographie, de résines pour matériaux composites et de masses d'étanchéité.

9. Composition de revêtement, contenant
a) un ou plusieurs liants contenant des groupes allophanate, durcissant sous l'effet d'un rayonnement selon la revendication 7,
b) le cas échéant un ou plusieurs polyisocyanates avec des groupes isocyanate libres ou bloqués, qui sont exempts de groupes réagissant avec polymérisation sous l'effet d'un rayonnement actinique avec des composés éthyléniquement insaturés,
c) le cas échéant d'autres composés différents de ceux de a), qui présentent des groupes réagissant avec polymérisation sous l'effet d'un rayonnement actinique avec des composés éthyléniquement insaturés et le cas échéant des groupes NCO libres ou bloqués,
d) le cas échéant un ou plusieurs composés réactifs avec isocyanate, contenant de l'hydrogène actif,
e) des initiateurs,
f) le cas échéant des solvants et
g) le cas échéant des adjuvants et des additifs.

10. Substrats revêtus par des revêtements pouvant être obtenus à partir de liants contenant des groupes allophanate, durcissant sous l'effet d'un rayonnement, selon la revendication 7.
